# EUROPEAN PATENT SPECIFICATION

(11) **EP 1 084 325 B1**
(45) Date of publication and mention of the grant of the patent: **23.10.2002**
(21) Application number: 99924064.1
(22) Date of filing: 25.05.1999
(51) Int. Cl.: E21B 21/00, E21B 17/00

(54) **ARRANGEMENT FOR THE REMOVAL OF CUTTINGS AND GAS ARISING FROM DRILLING OPERATIONS**
VORRICHTUNG ZUR ENTFERNUNG VON BEI BOHROPERATIONEN ANFALLENDEM BOHRKLEIN UND GAS
SYSTEME D'EVACUATION DES DEBLAIS ET DES GAZ PROVENANT D'OPERATIONS DE FORAGE

(30) Priority: 26.05.1998 NO 982394
(43) Date of publication of application: 21.03.2001
(73) Proprietor: AGR Services AS, 5363 Agotnes (NO)
(72) Inventor: EDVARDSEN, Per, Espen, N-5071 Loddefjord (NO)
(74) Representative: Jorritsma, Ruurd
(86) International application number: PCT/NO99/00165
(87) International publication number: WO 99/061745

(56) References cited:
- US-A- 1 811 761
- US-A- 2 082 329
- US-A- 2 730 333
- US-A- 3 901 332

## Description

The present invention relates to an arrangement mainly for the removal of bore cuttings, mud, clay and gas and the like in connection with top hole boring for preventing the accumulation of bore cuttings and the like around the mouth of the bore hole. The arrangement will also function as a " diverter ", that is to say a diversion system for gas. Gas represents a problem when during top hole boring with floating rigs in the first phases of drilling the sleeve pipes have not been set. With large quantities of gas during top hole boring the drilling must be terminated in order to avoid sinking of the rig because of reduced buoyancy. Since the arrangement according to the invention has a substantially fluid-tight connection for removing bore cuttings and the like the gas will also follow, with the result that the gas effluent is guided as required 100 - 300 metres from the rig, and thus does not represent a problem.

In connection with drilling on the ocean bottom bore cuttings are formed around the boring gauge (template). It is usual to employ remotely controlled underwater vehicles (ROV - " remote operated vehicle ") with cameras for monitoring and performance operations, and the bore cuttings/dust in the region around the mouth of the bore hole represent therefore a significant visibility problem. Bore cuttings are fragments of species of stone which during drilling are brought up with the drill sludge.

Norwegian Patent NO 302043 describes a submersible ejector-driven dredging apparatus which works on the sea bottom, and there bore cuttings are transferred via a hose from the mouth of the bore hole and are placed at another location on the bottom of the sea. This dredging apparatus is hoisted down from a drilling rig and is thus connected to the rig by both wire and electric cables.

US 2 082 329 A describes a conduit system guiding bore cuttings. However, this prior art document does not deal with a drilling rig from which a drill stem is guided to the ocean bottom.

The problem with accumulations of bore cuttings at the mouth of the bore hole is solved by the present invention in a simpler manner by employing rig pumps of the drilling rig for pressing out the bore cuttings to a conduit system. In order to make this possible an end piece if constructed which is arranged between drilling stem and sleeve pipe and which ensures in the main a fluid-tight seal between the sleeve pipe and the drill stem. The arrangement is equipped in that portion which faces towards the drill stem with elastic sealing means, for example rondelles.

The present invention which relates to a system for the removal of bore cuttings from the mouth of the bore hole, is thus characterised in that between the inner surface of the sleeve pipe and the outer surface of the drill stem is arranged an end piece which forms a seal, in the main a fluid-tight seal, between the sleeve pipe and the drill stem, and that at least one exit passage is arranged in the sleeve pipe which is directly connected with a conduit system which guides the bore cuttings and the like to a location which is at a distance from the mouth of the bore hole.

Specific embodiments of the arrangement are evident from the dependent claims 2 - 10.

An embodiment of the invention will now be further explained with reference to the accompanying Figures, where:
Fig. 1 shows in a general view a drilling rig ( which is heavily diminished relative to the remainder of the installation), and an arrangement according to the present invention for the removal of bore cuttings.
Fig. 2 shows how an end piece is positioned between sleeve pipe and drill stem, and how it is positioned relative to template and base unit. The Figure also shows the conduit system which guides the bore cuttings away from the mouth of the bore hole.
Fig. 3 is a section of the upper portions of the installation seen from above, and illustrates a jet line which is built into the installation.
Fig. 4 shows in more detail how the end piece according to the invention is designed, and particularly how the sealing functions on feeding of the drill stem downwards (" stripping in " ) and upwards ( " stripping out ") respectively.
Fig. 5 shows a simplified view of how the pressure from the well ensures a fluid-tight seal between the sleeve pipe and the drill stem.

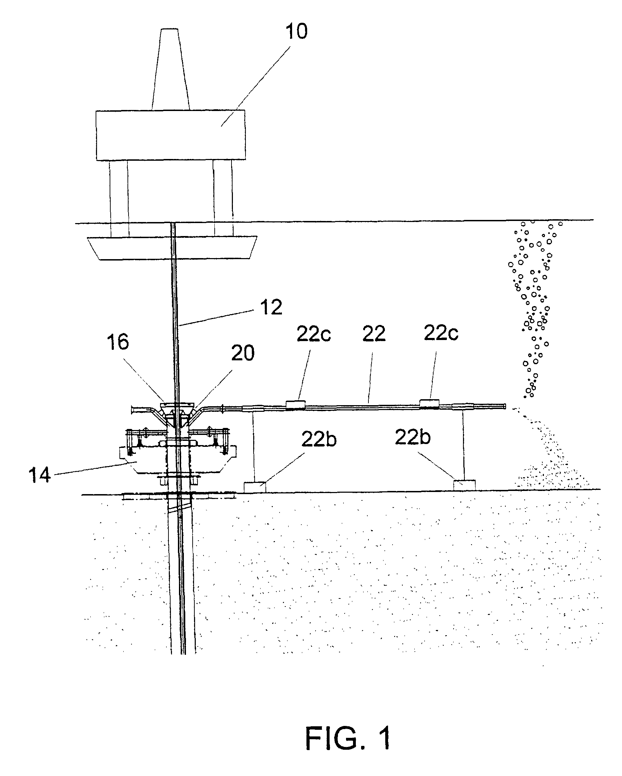

A floating drilling rig 10 is shown in Fig. 1 in a position above the ocean bottom. From the drilling rig 10 there extends a drill stem 12 to the ocean bottom. To the ocean bottom there is fastened a template (drilling gauge) 14. To the template (14) there is fastened a base unit (16) and a sleeve pipe (18). The drill stem 12 with associated drill crown (not shown) is guided through the sleeve pipe (18), centrally in the latter, for boring of the well. Further Fig. 1 shows an end piece (20) according to the invention, and a conduit system (22) which guide bore cuttings and consumed bore sludge to a location at a distance from the mouth of the bore hole. The end piece (20) also functions as a controlling and centering arrangement for correctly positioning the drill stem relative to the sleeve pipe (18), template (14) and base unit 16, at the same time as the end piece (20) ensures a substantially fluid-tight seal between the drill stem (12) and the sleeve pipe (18).

Since there is ensured in this manner a seal which is substantially fluid-tight between the sleeve pipe 18 and the drill stem 12, the pressure which is established by the supply of bore sludge, by means of the rig pumps to the bore hole, that is to say the pressure increase which is formed in the annular space 24 between the sleeve pipe and the drill stem 12, leads to the bore cuttings, gas and consumed bore sludge being able to be led out through a conduit system 22, illustrated here as a levelled effluent system, so that the bore cuttings are led away from the mouth of the bore hole. This involves the rig pumps being employable for removing bore sludge from the mouth of the bore hole, and thus there is used equipment which is already present on drilling platforms 10 to perform this task. The novel arrangement for removing bore cuttings in accordance with the invention has thus clear advantages relative to the known technique in that only two components, that is to say the end piece 20 and the conduit system 22, are to be mounted in the installation.

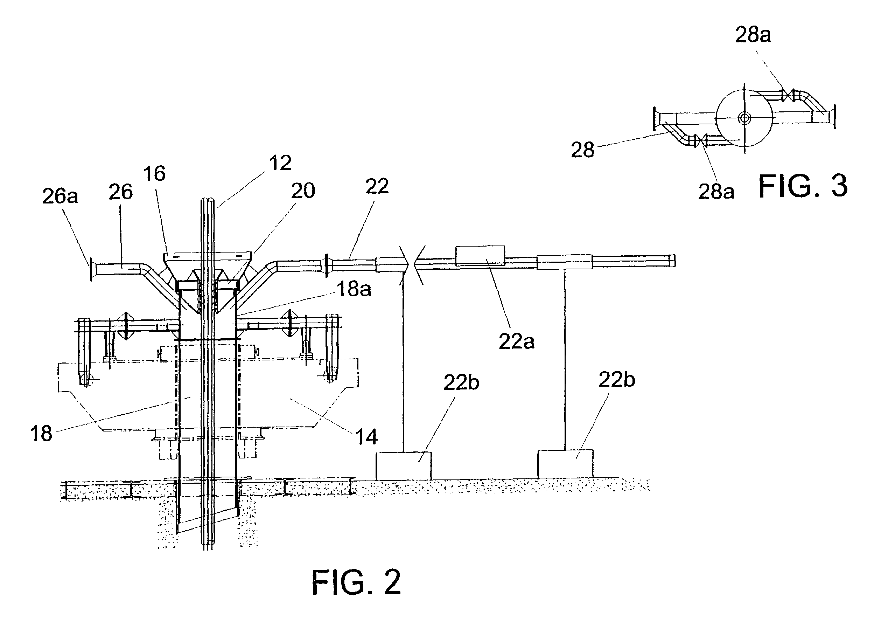

Fig. 2 shows in somewhat more detail how the end piece 20 is positioned between the sleeve pipe 18 and the drill stem 12. Since the drill crown has a diameter which is greater than the drill stem 12 there is formed a longitudinal annular space 24 between the inner surface of the sleeve pipe 18 and the outer surface of the drill stem 12. On drilling bore sludge is led via the drill stem 12 to the bore hole. Bore sludge is a liquid which is employed for a series of different functions, inter alia for maintaining control over the pressure in the well and for cooling and lubricating the drill crown, and consists as a rule of water, clay soil and chemicals. With the constant supply of bore sludge to the bore hole the pressure which is established (by the rig pumps) will lead to bore cuttings, which are formed during drilling, and consumed bore sludge and gas being led via the annular space 24 to the surface of the ocean bottom. In known solutions this waste will be guided between the sleeve pipe 18 and the drill stem 12 to above the upper end edge of the sleeve pipe 18. The solution according to the present invention bases itself on forming a seal between the sleeve pipe 18 and the drill stem 12, and further that there be established an exit passage in pipe wall 18a of the sleeve pipe 18 so that the bore cuttings and sludge and possible gases are led via this opening 18a and out into a conduit system 22. Bore sludge and cuttings and gas are thus led away from the mouth of the bore hole, and no longer represent a problem.

It is evident from Figs. 2 and 3 how a construction of the end piece 20 is designed. The end piece has an outer circular form having a diameter which is somewhat less than the inner diameter of the sleeve pipe 8 it is to be employed together with. The outer surface of the end piece can be provided with sealing means, such as rubber gaskets and the like. The end piece 20 has in the centre an opening 20a which is adapted to the drill stems 12 and associated sleeves 12a which it is to be employed together with, that is to say that the diameter of the opening is somewhat larger than the outer diameter of the sleeve of the drill stem. The inner surface of the opening 20a is provided with sealing means 20e which ensure a substantially fluid-tight seal between the sleeve pipe 18 and the drill stem 12. The flexibility/elasticity of the sealing means 20e is sufficient for the seal to become substantially fluid-tight both when the drill stem 12 is enclosed by the end piece 20 and when joint sleeves 12a are enclosed by the end piece.

In its lower portion the illustrated construction of the end piece 20 is further conically designed in that these surfaces function as guide surfaces 20b for on mounting guiding the end piece 20 into place in the sleeve pipe 18. The end piece 20 also has a portion 20c with a somewhat larger periphery than the remainder of the end piece in that this functions as a stop face against the upper end edge of the sleeve pipe 18. Further in its upper portion the end piece 20 is arranged with conical surfaces so that these function as guide surfaces 20d in connection with the penetration of the drill stem 12 in the end piece 20. By virtue of the design of the end piece 20 this will in addition to sealing between the sleeve pipe 18 and the drill stem 12, also ensure a favourable guiding and centering of the drill stem 12 relative to sleeve pipe 18, base unit 16, template 14 and bore hole.

In the sleeve pipe 18 there is arranged an opening 18a for exporting bore cuttings, - sludge and gas. Preferably this opening is placed so that this is suitably led via surfaces 20a of the end piece and directly into the conduit system 22, that is to say that the opening 18a is adjoining to the surface 20a and that the angle relative to the sleeve pipe 18 on the first portion 22a of the conduit system 22 corresponds to the angle for the surface 20a.

Fig. 2 shows the conduit system 22 for transferring of bore cuttings, - sludge and gas. The conduit system 22 is levelled in that it is equipped with anchoring elements 22b and floating elements 22c so that the major portion of the conduit system has a substantially horizontal dimension. The length of the conduit system 22 is adapted for the purpose, and is often of an order of magnitude of from 100 to 300 metres.

The arrangement also includes a " back up " line 26 which can be opened with valve 26a if the conduit system 22 is sealed shut. Further there is in the arrangement a jet line 28 (see Fig. 3) which makes it possible to supply via valves 28a a fluid of high pressure for detaching bore cuttings and - sludge which has been stucked in the system.

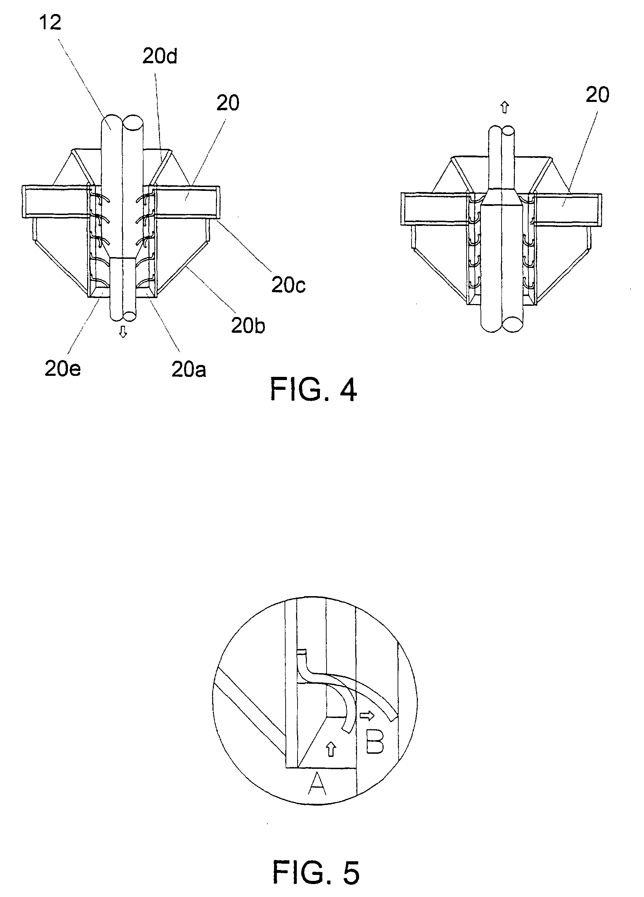

Fig. 3 shows an embodiment of the sealing means 20e where these are designed as a number of rondelles 20e which extend axially from the inner surface of the end piece 20 towards the centre of the opening 20a. The rondelles 20e are preferably constructed of nitrile rubber since this is both elastic and wear resistant. However other materials can be employed. The rondelles 20e can be less rigid in that portion which is in contact with the drill stem than in that portion which is fastened to the end piece 20, for example by the thickness of the rondelles 20e decreasing towards the centre. The rondelles 20e have a size and design which ensures a substantially fluid-tight coupling between the end piece 20 and drill stem 12 even if the diameter of the drill stem 12 varies. Fig. 5 illustrates in a section a rondelle 20e in contact with a drill stem 12. By virtue of the drill stem 12 being guided downwards relative to the end piece 20 the cross-section of the rondelle 20e will produce an S-like form. On guiding of the drill stem 12 in the opposite direction the outer portion of the rondelle is inverted and the rondelles receive a U-like cross-sectional form.

The inventive concept of the present invention is therefore that there is established a seal between the drill stem 12 and sleeve pipe 18, and that there is established an exit passage 18a for exporting bore sludge and - cuttings, since the pressure which is established in the annular space will thus forcibly guide bore sludge, - cuttings and gases via the opening 18a to a conduit system 22. In the same manner rig pumps of the drilling rig 10 can be employed for establishing the force which is necessary for transferring the bore cuttings to a location at a given distance from the mouth of the bore hole.

## Claims

1. Arrangement for the removal of bore cuttings which are formed by drilling of ocean floor formations, comprising a drilling rig (10) from which a drill stem (12) is guided to the ocean bottom for drilling thereof, the drill stem (12) being guided in the centre of a sleeve pipe (18) and base unit (16) which is anchored to the ocean bottom via template (14), and where between the inner surface of the sleeve pipe (18) and the outer surface of the drill stem (12), the drill crown having a larger periphery than the drill stem (12), there is formed relative to the drill stem (12) and the sleeve pipe (18) a longitudinal annular space through which bore cuttings, gases and consumed bore sludge are pressed as a result of the pressure which is established in the annular space as bore sludge is fed through the drill stem to the bore hole, **characterised in that** at the upper and edge of the sleeve pipe (18) between the inner surface of the sleeve pipe (18) and the outer surface of the drill stem (12) there is arranged an end piece (20) which forms a seal, in the main a fluid-tight seal, between the sleeve pipe (18) and the drill stem (12), and that there is arranged in the wall portion of the sleeve pipe (18) at least one exit passage (18a) which is directly connected with a conduit system (22) which guides bore cuttings, - sludge and gases to a location which is at a distance from the mouth of the bore hole.

2. Arrangement in accordance with claim 1, **characterised in that** the end piece (20) is conically designed in its lower portion this design forming a guide surface (20b) in the positioning of the end piece (20) between the sleeve pipe (18) and the drill stem (12).

3. Arrangement in accordance with claim 2, **characterised in that** the conical lower portion of the end piece (20) and the portion (22a) of the conduit system (22) which is coupled to the sleeve pipe (18) are arranged at approximately the same angle relative to the sleeve pipe (18), and at such a distance relative to each other that the concave lower portion of the end piece (20) forms guide surfaces for the bore cuttings.

4. Arrangement in accordance with one of the claims 1 - 3, **characterised in that** the end piece (20) in its upper portion in the inner surface of this portion is conically designed so that the oblique surfaces form guide surfaces (20d) in connection with the introduction of the drill stem (12) in the sleeve pipe (18).

5. Arrangement in accordance with one of the claims 1 - 4, **characterised in that** the end piece is in a portion designed with a flange portion (20c) which is adapted to be supported on the upper end portion of the sleeve pipe (18).

6. Arrangement in accordance with one of the claims 1 - 5, **characterised in that** the end piece (20) is provided along the inner surface with sealing means (20e) adapted so that there is ensured a substantially fluid-tight seal between the sleeve pipe (18) and the drill stem (12).

7. Arrangement in accordance with claim 6, **characterised in that** the sealing means (20e) is sufficiently elastic for ensuring a substantially fluid-tight seal between sleeve pipe (18) and drill stem (12) in portions of the drill stem having a small periphery and in portions of the drill stem having a large periphery, for example by a joint sleeve (12a).

8. Arrangement in accordance with claim 6 or 7, **characterised in that** the sealing means (20e) comprise an number of rondelles (20e) constructed of an elastic material.

9. Arrangement in accordance with claim 8, **characterised in that** by guiding the drill stems downwards the rondelles (20e) have in cross-section an S - like form where that portion which is in contact with the end piece (20) faces upwards and where that portion which is in contact with the drill stem (12) faces downwards.

10. Arrangement in accordance with one of the claims 8 - 9, **characterised in that** the rondelles (20e) **in that** portion which is in contact with the drill stem (12) is sufficiently elastic and exhibits a sufficient friction relative to the drill stem (12) so that the S-form of the rondelles, on guiding of the drill stem downwards, is converted to a U-like shape as the drill stem (12) is drawn upwards relative to the sleeve pipe (18).

## Patentansprüche

1. Vorrichtung zur Entfernung von Bohrklein, das durch Anbohren von Meeresbodenformationen gebildet ist, welche eine Bohranlage (10) umfaßt, von der aus ein Bohrgestänge (12) zum Meeresboden zum Anbohren desselben geführt ist, wobei das Bohrgestänge (12) in der Mitte eines Hülsenrohres (18) und einer Basiseinheit (16) gerührt ist, die im Meeresboden über Schablone (14) verankert ist, und wobei zwischen der Innenfläche des Hülsenrohres (18) und der Außenfläche des Bohrgestänges (12), wobei die Bohrkrone einen größeren Umfang aufweist als das Bohrgestänge (12), relativ zum Bohrgestänge (12) und zum Hülsenrohr (18) ein sich in Längsrichtung erstreckender Ringraum ausgebildet ist, durch den Bohrklein, Gase und verbrauchter Bohrschlamm als ein Ergebnis des Druckes durchgedrückt werden, der im Ringraum aufgebaut wird, wenn Bohrschlamm durch das Bohrgestänge zum Bohrloch zugeführt wird, **dadurch gekennzeichnet, daß** an der Kante des oberen Endes des Hülsenrohres (18) zwischen der Innenfläche des Hülsenrohres (18) und der Außenfläche des Rohrgestänges (12) ein Endstück (20) angeordnet ist, das eine Abdichtung, im wesentlichen eine fluiddichte Abdichtung, zwischen dem Hülsenrohr (18) und dem Bohrgestänge (12) bildet, und daß im Wandabschnitt des Hülsenrohres (18) wenigstens ein Auslaß (18a) angeordnet ist, der direkt verbunden ist mit einem Leitungssystem (22), das Bohrklein, Bohrschlamm und Gase zu einer Stelle leitet, die in einer gewissen Entfernung von der Öffnung des Bohrloches liegt.

2. Vorrichtung nach Anspruch 1, **dadurch gekennzeichnet, daß** das Endstück (20) in seinem unteren Abschnitt konisch ausgestaltet ist, wobei diese Ausgestaltung eine Leitfläche (20b) bei der Positionierung des Endstückes (20) zwischen dem Hülsenrohr (18) und dem Bohrgestänge (12) bildet.

3. Vorrichtung nach Anspruch 2, **dadurch gekennzeichnet, daß** der konische untere Abschnitt des Endstückes (20) und der Abschnitt (22a) des Leitungssystems (22), das mit dem Hülsenrohr (18) verbunden ist, in ungefähr demselben Winkel relativ zum Hülsenrohr (18) angeordnet sind und in solch einem Abstand zueinander, daß der konkave untere Abschnitt des Endstückes (20) Leitflächen für das Bohrklein bildet.

4. Vorrichtung nach einem der Ansprüche 1 bis 3, **dadurch gekennzeichnet, daß** das Endstück (20) in seinem oberen Abschnitt in der Innenfläche dieses Abschnittes konisch ausgestaltet ist, so daß die schrägen Oberflächen Leitflächen (20d) in Verbindung mit der Einführung des Bohrgestänges (12) in das Hülsenrohr (18) bilden.

5. Vorrichtung nach einem der Ansprüche 1 bis 4, **dadurch gekennzeichnet, daß** das Endstück in einem Abschnitt mit einem Flanschabschnitt (20c) ausgestaltet ist, der so angepaßt ist, daß er sich auf dem oberen Endabschnitt des Hülsenrohres (18) abstützt.

6. Vorrichtung nach einem der Ansprüche 1 bis 5, **dadurch gekennzeichnet, daß** das Endstück (20) entlang der Innenfläche mit Dichtungsmitteln (20e) versehen ist, die so angepaßt sind, daß eine im wesentlichen fluiddichte Abdichtung zwischen dem Hülsenrohr (18) und dem Bohrgestänge (12) sichergestellt ist.

7. Vorrichtung nach Anspruch 6, **dadurch gekennzeichnet, daß** die Dichtungsmittel (20e) ausreichend elastisch sind, um eine im wesentlichen fluiddichte Abdichtung zwischen Hülsenrohr (18) und Bohrgestänge (12) in Abschnitten des Bohrgestänges mit einem kleinen Umfang und in Abschnitten des Bohrgestänges mit einem großen Umfang, zum Beispiel durch eine Verbindungshülse (12a) sicherzustellen.

8. Vorrichtung nach Anspruch 6 oder 7, **dadurch gekennzeichnet, daß** die Dichtungsmittel (20e) eine Anzahl von Ringscheiben (20e) umfassen, die aus einem elastischen Material konstruiert sind.

9. Vorrichtung nach Anspruch 8, **dadurch gekennzeichnet, daß** durch Hindurchführen des Bohrgestänges nach unten die Rondelle (20e) im Querschnitt eine S-ähnliche Form aufweisen, wo derjenige Abschnitt, der in Kontakt mit dem Endstück (20) steht, nach oben zeigt und wo derjenige Abschnitt, der in Kontakt mit dem Bohrgestänge (12) steht, nach unten zeigt.

10. Vorrichtung nach einem der Ansprüche 8 bis 9, **dadurch gekennzeichnet, daß** die Ringscheiben (20e) in demjenigen Abschnitt, der in Kontakt mit dem Bohrgestänge (12) steht, ausreichend elastisch sind und eine so ausreichende Reibung relativ zum Bohrgestänge (12) zeigen, daß die S-Form der Ringscheiben, beim Hindurchführen des Bohrgestänges nach unten, in eine U-ähnliche Form überführt wird, wenn das Bohrgestänge (12) relativ zum Hülsenrohr (18) nach oben gezogen wird.

## Revendications

1. Système pour l'évacuation de déblais de forage formés en forant des formations de fonds océaniques, comprenant un appareil de forage (10) depuis lequel une tige de forage (12) est guidée vers le fond de l'océan pour y forer, la tige de forage (12) étant guidée au centre d'un manchon tubulaire (18), et une base (16) accrochée au fond de l'océan par l'intermédiaire d'un châssis de guidage (14), et où, entre la surface intérieure du manchon tubulaire (18) et la surface extérieure de la tige de forage (12), la couronne de forage ayant un plus grand pourtour que la tige de forage (12), il est formé par rapport à la tige de forage (12) et au manchon tubulaire (18) un espace longitudinal annulaire dans lequel les déblais de forage, les gaz et les boues de forage consommées sont comprimés par suite de la pression établie dans l'espace annulaire à mesure que des boues de forage sont amenées dans la tige de forage jusqu'au trou de forage, **caractérisé en ce que**, sur le bord de l'extrémité supérieure du manchon tubulaire (18), entre la surface intérieure du manchon tubulaire (18) et la surface extérieure de la tige de forage (12), est disposé un embout (20) qui forme un joint, principalement un joint étanche aux fluides, entre le manchon tubulaire (18) et la tige de forage (12), et **en ce que** dans la paroi du manchon tubulaire (18) est ménagé au moins un passage de sortie (18a) directement relié à un système de conduit (22) qui guide les déblais de forage, boues et gaz jusqu'à un site distant de l'embouchure du trou de forage.

2. Système selon la revendication 1, **caractérisé en ce que** l'embout (20) a une forme conique dans sa partie inférieure, cette forme créant une surface de guidage (20b) lors de la mise en place de l'embout (20) entre le manchon tubulaire (18) et la tige de forage (12).

3. Système selon la revendication 2, **caractérisé en ce que** la partie inférieure conique de l'embout (20) et la partie (22a) du système de conduit (22) accouplée avec le manchon tubulaire (18) sont disposées approximativement suivant le même angle par rapport au manchon tubulaire (18), et à une distance l'une de l'autre telle que la partie inférieure concave de l'embout (20) forme des surfaces de guidage pour les déblais de forage.

4. Système selon l'une quelconque des revendications 1 à 3, **caractérisé en ce que** l'embout (20) a une partie supérieure dont la surface intérieure a une forme conique de façon que les surfaces obliques créent des surfaces de guidage (20d) lors de l'introduction de la tige de forage (12) dans le manchon tubulaire (18).

5. Système selon l'une quelconque des revendications 1 à 4, **caractérisé en ce que** l'embout est une partie pourvue d'une partie formant bride (20c) conçue pour être supportée sur la partie d'extrémité supérieure du manchon tubulaire (18).

6. Système selon l'une quelconque des revendications 1 à 5, **caractérisé en ce que** l'embout (20) comporte sur sa surface intérieure un moyen d'étanchéité (20e) conçu de façon à réaliser un joint sensiblement étanche aux fluides entre le manchon tubulaire (18) et la tige de forage (12).

7. Système selon la revendication 6, **caractérisé en ce que** le moyen d'étanchéité est suffisamment élastique pour assurer un joint sensiblement étanche aux fluides entre le manchon tubulaire (18) et la tige de forage (12) dans des parties de la tige de forage ayant un petit pourtour et dans des parties de la tige de forage ayant un grand pourtour, par exemple par un manchon d'assemblage (12a).

8. Système selon la revendication 6 ou 7, **caractérisé en ce que** le moyen d'étanchéité (20e) comporte un certain nombre de rondelles (20e) en matière élastique.

9. Système selon la revendication 8, **caractérisé en ce que**, en guidant les tiges de forage vers le bas, les rondelles (20e) ont, en coupe, une forme en S, la partie au contact de l'embout (20) étant orientée vers le haut et la partie au contact de la tige de forage (12) étant orientée vers le bas.

10. Système selon l'une des revendications 8 et 9, **caractérisé en ce que** les rondelles (20e), dans la partie au contact de la tige de forage (12), sont suffisamment élastiques et présentent un frottement suffisant par rapport à la tige de forage (12) pour que la forme en S des rondelles, lors du guidage de la tige de forage vers le bas, soit convertie en une forme en U lorsque la tige de forage (12) est tirée vers le haut par rapport au manchon tubulaire (18).
